# FASCICULE DE BREVET EUROPEEN

(11) **EP 3 829 626 B1**
(45) Date de publication et mention de la délivrance du brevet: **02.08.2023**
(21) Numéro de dépôt: 19742046.6
(22) Date de dépôt: 19.07.2019
(51) Int. Cl.: A61K 38/48, A61P 17/14

(54) **EXTRAIT DE LESPEDEZA CAPITATA POUR SON UTILISATION DANS LE DOMAINE CAPILLAIRE**
LESPEDEZA-CAPITATA-EXTRAKT ZUR VERWENDUNG AUF DEM GEBIET DER HAARPFLEGE
LESPEDEZA CAPITATA EXTRACT FOR USE IN THE FIELD OF HAIR CARE

(30) Priorité: 27.07.2018 FR 1856999
(43) Date de publication de la demande: 09.06.2021
(73) Titulaire: Pierre Fabre Dermo-Cosmétique, 92100 Boulogne-Billancourt (FR)
(72) Inventeur: LETI, Mathieu, 31450 MONTGISCARD (FR); DAUNES-MARION, Sylvie, 31000 TOULOUSE (FR); LEVEQUE, Marguerite, 31400 TOULOUSE (FR)
(74) Mandataire: Regimbeau
(86) Numéro de dépôt international: PCT/EP2019/069569
(87) Numéro de publication internationale: WO 2020/020791

(56) Documents cités:
- EP-A1- 1 226 827
- WO-A1-97/28814
- WO-A2-2018/127612
- CN-A- 103 251 533
- KR-A- 20130 091 406
- US-A- 4 460 578
- US-A1- 2013 078 202

## Description

### DOMAINE TECHNIQUE

La présente invention concerne un extrait de *Lespedeza capitata* Michx. pour son utilisation en dermatologie dans le domaine capillaire ; plus particulièrement dans le traitement et/ou la prévention de l'alopécie et/ou de la séborrhée du cuir chevelu.

### ETAT DE LA TECHNIQUE ANTERIEURE

*Lespedeza capitata* Michx. est une herbacée vivace de la famille des Leguminosae originaire d'Amérique du Nord. Il existe des cultures de *Lespedeza capitata* Michx. en Europe et notamment en France.

*Lespedeza capitata* Michx. est une plante tomenteuse dont les tiges dressées peuvent atteindre une hauteur de 1,5 mètres. Ses feuilles sont alternes, composées, généralement trifoliées ; les folioles mesurent environ 4,5 x 1,8 cm ; le pétiole est court (2-5 mm). Les feuilles et les tiges sont couvertes de poils apprimés pouvant donner à la plante un reflet argenté. Les fleurs, de type papilionacé, sont densément groupées en inflorescences axillaires. Les pétales sont blancs à l'exception de l'étendard qui présente une tâche rose à violette. Le calice est persistant et se pare d'une teinte brune avec le temps. Les fruits sont indéhiscents et portent une seule graine. *Lespedeza capitata* Michx. s'enracine profondément, avec des racines principales pouvant creuser jusqu'à 2,5 mètres dans le sol.

*Lespedeza capitata* Michx. fait partie de la médecine traditionnelle de plusieurs ethnies amérindiennes. Les Omaha et les Ponça utilisaient les tiges en tant que moxa pour traiter les rhumatismes ou la névralgie. Les Comanches utilisaient les feuilles sous forme d'infusion. Les Meskwaki utilisaient la racine comme antidote en cas d'empoisonnement (USDA-NRCS. Plant Guide 05 dec. 2000). Aujourd'hui, le *Lespedeza capitata* Michx. est principalement cultivé pour son utilisation dans des compléments alimentaires, des préparations pharmaceutiques, homéopathiques et vétérinaires.

Les parties aériennes de *Lespedeza capitata* Michx. ont été essentiellement étudiées pour leurs propriétés dans la sphère urinaire (pouvoir diurétique et antiazotémique notamment). Plusieurs études cliniques sont documentées, la plupart ont été réalisées dans les années 60 à 80, en France principalement. Les études cliniques portent notamment sur l'administration d'extraits de *Lespedeza capitata* Michx. par voie intraveineuse à des patients souffrant d'insuffisance rénale. L'activité hypocholestérolémiante de la teinture de *Lespedeza capitata* Michx. administrée par voie orale a été mise en évidence lors d'une étude clinique menée sur 39 sujets atteints d'hypercholestérolémies stables (Yarnell and Abascal, Alternative and Complementary Thérapies. 13(1) : 18-23, 2007).

Aujourd'hui, les parties aériennes de *Lespedeza capitata* Michx. sont toujours utilisées pour leurs propriétés thérapeutiques. Réduites en poudre, ou sous forme d'extraits, elles sont commercialisées pour la préparation d'infusions, sous forme de compléments alimentaires, de préparations homéopathiques, pharmaceutiques (hyperazotemie ; sphère urinaire) ou vétérinaires (sphère urinaire).

Une publication récente montre que l'extrait éthanolique de *Lespedeza capitata* Michx. induit *in vitro* la prolifération des fibroblastes et des kératinocytes, une inhibition de la collagénase, la stimulation de la synthèse de collagène par les fibroblastes, et une action lipolytique (Pastorino et al. 2017. Industrial Crops and Products. 96 : 158-164). Il est par ailleurs revendiqué dans la demande de brevet CN104606122 que le Lespedeza capitata, en mélange avec d'autres plantes, présente la capacité de brûler les graisses abdominales liées à une grossesse.

### RESUME DE L'INVENTION

De façon inattendue et surprenante, les demandeurs ont mis en évidence qu'un extrait de *Lespedeza capitata* Michx. présente des activités pharmacologiques d'intérêt dans le domaine du traitement et/ou de la prévention des troubles dermo-capillaires et notamment pour lutter contre la chute de cheveux ainsi que pour traiter la séborrhée du cuir chevelu et les troubles associés.

En effet, les inventeurs ont mis en évidence qu'un extrait de *Lespedeza capitata* Michx. inhibe la synthèse et la libération de la protéine Dickkopf-related protein 1 (DKK1) par les cellules de la papille dermique. Ainsi, l'extrait de *Lespedeza capitata* Michx. va permettre de retarder et prévenir la chute de cheveux et prolonger le cycle de vie du cheveu.

Par ailleurs, les inventeurs ont montré qu'un extrait de *Lespedeza capitata* Michx. inhibe l'activité de la 5α-réductase dans des cellules de la papille dermique. Par conséquent, l'extrait de *Lespedeza capitata* Michx. va être utile dans le domaine de l'antichute capillaire mais également dans la séborrhée du cuir chevelu et les troubles dermo-capillaires associés.

Cette nouvelle valorisation de *Lespedeza capitata* Michx. dans le domaine capillaire fait l'objet de la présente invention et permet d'envisager la déclinaison de différents produits à base de *Lespedeza capitata* Michx. aussi bien pour améliorer l'apparence esthétique du cheveu, en soin capillaire, que pour prévenir et/ou traiter la chute de cheveu et/ou la séborrhée du cuir chevelu et tous les troubles dermo-capillaires associés.
L'invention concerne donc un extrait de Lespedeza capitata pour son utilisation pour le traitement et/ou la prévention d'au moins un trouble dermo-capillaire, caractérisé en ce que le au moins trouble dermo-capillaire est choisi parmi l'alopécie, la séborrhée du cuir chevelu, ainsi que leurs combinaisons et que l'extrait est obtenu à partir d'une ou plusieurs parties de plante *Lespedeza capitata* choisie parmi les parties aériennes telles que tiges, branches, feuilles, fruits, graines et/ou fleurs, suite à une extraction par solvant hydrophile ou moyennement polaire choisi dans le groupe constitué des alcools en C1 à C5, des esters d'alkyles et un mélange hydro-alcoolique.

### DESCRIPTION DETAILLEE

Le soin des cheveux, non seulement à des fins cosmétiques mais aussi pour empêcher leur chute, pour les regénérer a toujours sollicité l'esprit de recherche.

Le follicule pileux est un mini-organe ancré dans la peau jusqu'à l'hypoderme, et dont la fonction principale est la production d'une tige pilaire. Le follicule pileux est une structure dynamique qui produit le cheveu. Les cheveux ne poussent pas continuellement mais selon un cycle de croissance pilaire divisé en 3 phases :
- Une phase de croissance (anagène), les cellules de la papille dermique (fibroblastes) envoient un signal aux cellules souches du bulbe qui permet leur prolifération. Ces cellules vont se transformer et envelopper la papille dermique pour former la matrice soufrée du cheveu. Elles se divisent et se différencient en kératinocytes, cellules responsables de la structure du cheveu. Pour que le cheveu soit bien structuré, les kératinocytes ont besoin de protéines soufrées, de vitamine B6 et de différents minéraux comme le zinc, le magnésium. La durée de cette phase détermine la longueur du cheveu et dépend de la prolifération et de la différenciation des cellules de la matrice à la base du follicule.
- Une phase de régression (catagène), la matrice meurt et de ce fait la papille dermique n'est plus au contact avec cette matrice. Il n'y a plus d'échange entre les cellules. Le follicule et la papille dermique remontent vers l'épiderme.
- Une phase de repos (télogène), les cellules de la papille dermique et du bulbe sont intactes et inactives. Le cheveu tombe. Pour qu'un nouveau cheveu se développe, il faut que le cycle soit réinitié.

La chevelure se renouvelle donc en permanence et sur les 100 000 à 150 000 cheveux que comporte une chevelure, la majorité est en phase de croissance. Il existe une perte normale et physiologique de cheveux de l'ordre de 60 à 100 par jour pour une chevelure saine. Au-delà, la chute est dite pathologique qu'elle soit occasionnelle ou installée.

Le terme alopécie désigne la perte partielle ou générale des cheveux. De nombreux facteurs peuvent être impliqués dans l'alopécie comme par exemple les facteurs génétiques, l'âge, le sexe, les maladies, le stress, les problèmes hormonaux, les effets secondaires de médicaments, les cicatrices. Il est possible de distinguer plusieurs formes d'alopécie :
- L'alopécie androgénétique héréditaire, elle est la plus fréquente. La chute précoce des cheveux survient chez des sujets prédisposés génétiquement et elle atteint en particulier les hommes. Elle se manifeste par une diminution du volume des cheveux, voire une calvitie et touche 50% des hommes âgés de plus de 50 ans ;
- L'alopécie post-ménopausique, il s'agit de la plus fréquente cause de la calvitie chez la femme. Chez la femme, la chute des cheveux est plus diffuse et étendue que chez l'homme. L'alopécie diffuse féminine est un désordre qui démarre souvent à la ménopause et qui concerne environ 40% des femmes âgées de plus de 70 ans. Le terme diffus illustre que, contrairement à l'homme, la perte des cheveux concerne la totalité du cuir chevelu, de manière homogène ;
- L'alopécie aiguë ou réactionnelle, elle peut être liée à un traitement par chimiothérapie, un stress, un accouchement, des carences alimentaires importantes, une carence en fer, des troubles hormonaux, il s'agit d'une chute simultanée et diffuse d'une quantité importante de cheveux ;
- L'alopécie cicatricielle, elle peut être provoquée par des problèmes de peau (tumeur, brûlure, pelade), une irradiation aiguë, au lupus érythémateux ou des parasites (teigne, lichen) ;
- L'alopécie areata, elle semble être d'origine auto-immune et se caractérise par une atteinte en plaques plus ou moins larges et à un ou plusieurs endroits ;
- L'alopécie congénitale, rare, elle correspond à une absence de racines ou à des anomalies du cheveu (mutations).

L'alopécie est essentiellement liée à une perturbation du renouvellement capillaire qui entraine, dans un premier temps, l'accélération de la fréquence des cycles aux dépens de la qualité des cheveux puis de leur quantité. Le phénomène le plus fréquent est une réduction du cycle de croissance (phase anagène) dû à un arrêt de la prolifération cellulaire. La conséquence est une induction prématurée de la phase catagène et un nombre plus important de follicules pileux en phase télogène et donc une chute plus importante des cheveux. Pour lutter contre l'alopécie, il est donc nécessaire de relancer le cycle pilaire, par exemple, en activant la phase anagène.

Il est connu à ce jour que les mécanismes responsables de l'alopécie androgénétique héréditaire (que l'on appelait avant alopécie séborrhéique) mettent en jeu entre autres la composante hormonale avec la surexpression du récepteur aux androgènes (récepteur de la Testostérone et de la DHT) et une activité plus intense de l'enzyme 5 alpha-réductase. Cette dérégulation hormonale entraîne une production excessive de dihydrotestostérone (DHT), métabolite actif de la testostérone. Au niveau de la papille dermique, ce métabolite va stimuler la production d'inhibiteurs du cycle pilaire entraînant un raccourcissement de la phase anagène forçant le cheveu à passer trop vite en phase télogène et ne laissant pas au follicule pileux assez de temps pour fabriquer une kératine de qualité et nécessairement après plusieurs cycles, un épuisement de la capacité du follicule pileux à produire une tige pilaire.

Cette alopécie due à un excès d'androgènes touche également les femmes au moment de la ménopause (alopécie post-ménopausique) ou à la suite d'un traitement par des androgènes. Elle commence au niveau des tempes et à la couronne. Cette chute de cheveux est plus diffuse et étendue que chez l'homme. La perte de cheveux concerne la totalité du cuir chevelu, de manière homogène.

Un actif inhibiteur de 5 alpha réductase permet ainsi de traiter et/ou prévenir la chute de cheveux chez l'homme et/ou la femme.

Quant à la séborrhée, elle correspond à une production excessive de sébum par les glandes sébacées. Globalement, l'homme possède 2 000 000 de glandes sébacées annexées à 6 000 000 de poils et cheveux. La répartition des glandes sébacées est non uniforme. La densité des glandes sébacées atteint 300 à 900 glandes sébacées/cm² au niveau du visage et du cuir chevelu, et cette densité est de l'ordre de 100 glandes sébacées/cm² dans la partie haute du thorax et du dos. L'activité de la glande sébacée est influencée par les androgènes. Les androgènes ne sont actifs que sous l'influence de l'enzyme 5-alpha réductase qui assure la métabolisation des androgènes au niveau la glande sébacée ce qui induit la production de sébum. Une hyperactivation de l'enzyme 5-alpha réductase provoque la séborrhée.

Le siège des manifestations de la séborrhée est la région médio-faciale (front, nez menton) où les glandes sébacées sont les plus nombreuses et les plus volumineuses. La séborrhée se manifeste également au niveau du cuir chevelu où elle prédomine dans les régions frontale, fronto-temporale et au sommet du crâne.

La séborrhée du cuir chevelu occasionne des désagréments d'ordre esthétique et dermatologiques tels que la dermite séborrhéique du cuir chevelu et l'érythème du cuir chevelu.

La dermite séborrhéique (DS) du cuir chevelu se caractérise par des plaques inflammatoires roses et diffuses, recouvertes de squames-croûtes grasses, blanchâtres, adhérant à l'épiderme, parfois purulentes et provoquant souvent de fortes démangeaisons.

La séborrhée est souvent associée à l'alopécie androgénétique.

Un actif inhibiteur de l'activité de l'enzyme 5-α réductase permet donc de réduire la sécrétion de sébum, traiter la séborrhée et résoudre les désagréments d'ordre dermatologiques et/ou esthétique liés à la séborrhée.

Le cycle de croissance pilaire est un processus fortement régulé au cours duquel les différents compartiments du follicule pileux (papille dermique, cellules souches résidant dans le bulbe, kératinocytes de la matrice du follicule pileux) entretiennent des relations étroites à travers de nombreux échanges moléculaires. Parmi les signaux impliqués, le signal Wnt/β-caténine est connu pour favoriser la croissance des cheveux (Leiros et al., Br. J. Dermatol. 166(5)-1035-1042, 2012).

L'alopécie androgénétique, qui est la forme la plus commune de perte de cheveux, est clairement une maladie androgénique. Les androgènes dérégulent les facteurs sécrétés par la papille dermique, ce qui entraîne un défaut de différenciation des cellules souches par inhibition de la voie de signalisation Wnt canonique.
L'inhibiteur du cycle de croissance des cheveux « Dickkopf-related protein 1 (DKK1) » est connu pour bloquer l'activation canonique de la voie Wnt médiée par la β-caténine et favorise la progression de la phase catagène (Kwack et al., J. Invest. Dermatol. 132(6) :1554-60, 2012). DKK1 est également décrit comme étant régulé par les androgènes Kwack et al., J. Invest. Dermatol. 128(2) :262-9, 2008).
Pris ensemble, ces résultats montrent que DKK1 joue un rôle important dans la suppression induite par les androgènes de la croissance des cheveux et l'induction précoce de la phase catagène dans l'alopécie androgénétique (Inui and Itami, J. Dermatol. Sci. 61(1) :1-6, 2011).

### EXTRAIT SELON L'INVENTION

Dans la présente description, la plante *Lespedeza capitata* Michx. pourra être désignée de manière abrégée par le terme Lespedeza capitata.

Dans le cadre de la présente invention, l'extrait de Lespedeza capitata est obtenu à partir d'une ou plusieurs parties de plante Lespedeza capitata choisie parmi les parties aériennes telles que tiges, branches, feuilles, fruits, graines et/ou fleurs. De manière avantageuse il s'agit des parties aériennes choisies parmi les feuilles, les tiges, les fleurs, seules ou en mélange.

Dans un mode de réalisation particulier de l'invention, l'extrait est obtenu à partir de feuilles et/ou tiges et/ou fleurs de Lespedeza capitata, particulièrement sèches.

Selon un mode particulier, l'extrait est un extrait hydro-alcoolique, en particulier un extrait hydro-éthanolique. Avantageusement il s'agira d'un extrait issu d'une extraction hydro-alcoolique, en particulier hydro-éthanolique.

Selon un mode de réalisation préféré, l'extrait selon l'invention est susceptible d'être obtenu par un procédé selon l'invention décrit ci-après.

La plante ou partie de plante Lespedeza capitata peut être fraiche ou sèche, entière, coupée ou broyée puis soumise à une étape d'extraction.

Un procédé de préparation d'un extrait selon l'invention comprend une étape d'extraction de tout ou partie de plante Lespedeza capitata par un solvant hydrophile ou moyennement polaire choisi dans le groupe constitué des alcools en C1 à C5, des esters d'alkyles et un mélange hydro-alcoolique.

Par « solvant moyennement polaire » on entend, au sens de la présente invention, un solvant choisi dans le groupe constitué des alcools en C1 à C5, des esters d'alkyles (tels qu'acétate d'éthyle, acétate d'isopropyle en particulier), de mélanges hydro-alcooliques.

Par « esters d'alkyles », on entend au sens de la présente invention un composé R₁-COO-R₂, dans lequel R₁ et R₂ sont des groupements (C₁-C₆)alkyle, identiques ou différents. L'ester peut en particulier être un acétate, c'est-à-dire un composé CH₃COO-R₂.

Par groupement « (C₁-C₆)alkyle », on entend, au sens de la présente invention, une chaîne hydrocarbonée saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par « solvant hydrophile », on entend un solvant choisi dans le groupe constitué des alcools miscibles à l'eau (par exemple éthanol), et des mélanges de ceux-ci avec l'eau.

Par « extrait sec », on entend, au sens de la présente invention, un extrait dépourvu de solvant d'extraction ou de support, ou en contenant uniquement à l'état de trace non significative. Un tel extrait sec contient ainsi uniquement de la matière issue de *Lespedeza capitata.* Il peut contenir également des traces non significatives de solvant d'extraction.

Dans un mode de réalisation préféré de l'invention, le solvant d'extraction pourra être choisi parmi l'acétate d'éthyle, l'acétate d'isopropyle, un alcool de C1 à C5. Préférentiellement il s'agira d'un mélange alcool/eau. Avantageusement, il s'agira d'un mélange éthanol/eau.

Encore plus avantageusement, ce mélange éthanol/eau sera caractérisé par une proportion éthanol / eau de 9 : 1 à 7 : 3 (v/v).

Encore plus avantageusement, le solvant moyennement polaire est un mélange éthanol / eau dans la proportion 9 : 1 (v/v).

Selon un autre mode de réalisation particulier de l'invention, l'extraction est réalisée sous agitation ou de façon statique, à reflux, à température ambiante, ou à une température comprise entre la température ambiante et le reflux. Elle peut être assistée par ultra-sons, par micro-ondes ou par extrusion, dans un ratio poids de plantes / volume de solvant pouvant varier de 1 / 3 à 1 / 30, pendant une durée de 1 minute à 48 heures. L'extraction peut être renouvelée 2 à 3 fois.

Selon un autre mode de réalisation particulier de l'invention, la phase solide est ensuite séparée par centrifugation ou filtration afin de récupérer une phase liquide limpide exempte de particules. La filtration peut être réalisée sur papier filtre ou plaque de filtration présentant un seuil de coupure compris entre 5 et 20 µm, particulièrement entre 10 et 20 µm. Des filtres ou plaques de filtration adaptés peuvent être choisis parmi la gamme de plaques de filtration en profondeur série K de chez Pall^{®}. De telles plaques sont composées d'un mélange équilibré de fibres de cellulose, de terres de diatomées, et de perlite, ce qui permet la création d'une matrice bien définie. On pourra choisir ainsi des plaques Pall^{®} série K de type K300 à K900 par exemple. La phase liquide représentant l'extrait peut être plus ou moins concentrée pouvant aller jusqu'à l'obtention d'un extrait sec.

Dans un autre mode de réalisation de l'invention, un support peut être rajouté lors de l'étape de concentration de façon à obtenir un extrait contenant 1 à 75% d'extrait sec. Le support peut être de la maltodextrine, du lactose, de la silice, de la glycérine, un glycol, ou tout autre support cosmétologiquement acceptable et solubilisant l'extrait, préférentiellement d'origine biosourcée comme par exemples des glycols biosourcées (1,2-pentanediol ; 1,3-butanediol ; 1,3-propanediol...), et également les hydrotropes comme par exemple les alkyls glycosides (Sepiclear, Apyclean, APXC4...).

Selon un mode de réalisation particulier de l'invention, l'extrait peut être décoloré, par exemple sur charbon actif, de façon à éliminer tout ou partie des chlorophylles.

Selon un mode de réalisation particulier de l'invention, l'extrait de Lespedeza capitata selon l'invention est caractérisé par une teneur de 2 à 40 %, particulièrement de 5 à 35%, plus particulièrement de 10 à 30%, voire encore plus particulièrement environ 25%, en polyphénols totaux (% en poids par rapport au poids de l'extrait sec) exprimés en grammes d'épicatéchine pour 100 grammes d'extrait sec.

Par « environ », on entend dans la présente description que la valeur concernée peut être inférieure ou supérieure de 10%, notamment de 5%, en particulier de 1%, à la valeur indiquée.

### Composition selon l'invention

Un autre objet de l'invention concerne une composition dermatologique ou dermo-capillaire pour son utilisation pour le traitement et/ou la prévention d'au moins un trouble dermo-capillaire choisi parmi l'alopécie, la séborrhée du cuir chevelu, et leur combinaison comprenant au moins un extrait de *Lespedeza capitata* selon l'invention avec au moins un excipient dermatologiquement acceptable.

Dans la présente invention, on entend désigner par « dermatologiquement acceptable » ce qui est utile dans la préparation d'une composition dermatologique ou dermo-capillaire, qui est généralement sûre, non toxique et ni biologiquement ni autrement non souhaitable et qui est acceptable pour une utilisation dermatologique ou dermo-capillaire, notamment par application topique au niveau des cheveux et/ou du cuir chevelu.

Par « application topique », on entend une application sur la peau, notamment le cuir chevelu, les muqueuses, et/ou les phanères, notamment sur les cheveux et le cuir chevelu.

Par « phanère », on entend désigner, les cheveux, les poils, les sourcils, les cils, et/ou les ongles, préférentiellement les cheveux.

Dans un mode de réalisation particulier de l'invention, la composition selon l'invention est caractérisée en ce qu'elle se présente sous une forme adaptée à une administration topique au niveau du cuir chevelu et/ou des cheveux.

Dans un mode de réalisation particulier de l'invention, les compositions selon l'invention sont destinées à une application topique, notamment par application de la composition sur le cuir chevelu et/ou les cheveux et les phanères.

Les compositions selon l'invention pourront ainsi se présenter sous les formes qui sont habituellement connues pour une administration topique, c'est-à-dire notamment les lotions, les baumes, les shampoings, les mousses, les gels, les dispersions, les émulsions, les sprays, les sérums, les masques ou les crèmes, avec des excipients permettant notamment une pénétration afin d'améliorer les propriétés et l'accessibilité des principes actifs.

Avantageusement, les compositions selon l'invention peuvent se présenter sous les formes qui sont habituellement connues pour une administration topique sur les cheveux et le cuir chevelu, c'est-à-dire notamment un shampoing, un après-shampoing, une crème capillaire, une lotion capillaire, un masque ou un spray sans rinçage. Il s'agit ainsi de compositions cosmétiques, dermatologiques ou dermo-capillaires.

On distingue ainsi des produits formulés pouvant être rincés et des produits formulés ne nécessitant pas de rinçage.

Dans un mode de réalisation particulier de l'invention, la composition selon l'invention est caractérisée en ce qu'elle se présente sous une forme adaptée à une administration orale. Il a en effet été montré que Lespedeza capitata est capable d'inhiber l'enzyme de conversion du système rénine-angiotensine, même lorsqu'il est administré oralement (Yarnell, 2012, IJKD, vol 6, 407-418), cet extrait n'est donc pas complètement métabolisé par son passage gastro-intestinal.

Selon un autre mode de réalisation de l'invention, les compositions selon l'invention pourront aussi se présenter sous les formes qui sont habituellement connues pour une administration orale, c'est-à-dire notamment les comprimés, les gélules, les poudres, les granules et les solutions ou suspensions orales. Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique, la silice ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Ces compositions contiennent généralement, outre l'extrait selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents hydratants ou des eaux thermales, etc.

Dans un mode de réalisation, les compositions dermatologiques, en particulier dermo-capillaires, selon la présente invention comprendront de 0,0001% à 2%, de préférence 0,001 à 1% en poids, de manière préférée 0,005% à 0,8% en poids, de manière encore préférée 0,01% à 0,5% d'extrait de Lespedeza capitata, en poids d'extrait sec par rapport au poids total de la composition.

Les extraits de Lespedeza capitata utilisés dans les compositions selon l'invention comprendront avantageusement une teneur d'extrait sec de 1% à 10%, de préférence de 1% à 5%, en poids par rapport au poids total de l'extrait.

Dans un mode de réalisation particulier l'extrait de Lespedeza capitata est le seul agent actif utilisé dans la composition destinée au soin des cheveux et/ou du cuir chevelu. Par l'expression agent actif on entend un agent curatif ou préventif du ou des troubles dermo-capillaires visés par l'invention.

L'extrait selon l'invention ou la composition dermatologique selon l'invention peut aussi être utilisée en combinaison avec un traitement de l'alopécie, comme le finastéride ou le minoxidil, et/ou en combinaison avec des composés utiles pour une bonne structure du cheveu, comme par exemple des protéines soufrées, de la vitamine B6 et différents minéraux comme le zinc et/ou le magnésium.

L'extrait ou la composition selon l'invention pourra aussi être combiné avec au moins un agent anti-bactérien, un agent anti-fongique, un anti-inflammatoire ainsi que leurs mélanges.

L'extrait selon l'invention ou la composition dermatologique selon l'invention peut être utilisée chez un individu qui a subi une micro greffe.

De préférence, la composition selon l'invention a une texture légère permettant en outre une pénétration optimale sans graisser les cheveux et/ou les poils, ni le cuir chevelu. Dès les premières applications, la chevelure retrouvera force et vitalité. L'invention vise encore une composition dermatologique selon l'invention pour son utilisation pour le traitement et/ou la prévention d'au moins un trouble dermo-capillaire choisi parmi l'alopécie, la séborrhée du cuir chevelu et leurs combinaisons.
La composition selon l'invention pour le traitement et/ou la prévention d'au moins un trouble dermo-capillaire est caractérisé en ce que l'alopécie est choisie dans le groupe constitué par l'alopécie androgénétique, l'alopécie réactionnelle, l'alopécie post-ménopausique et l'alopécie aerata.

Un autre objet de la présente invention concerne une composition dermatologique comprenant un extrait de Lespedeza capitata selon l'invention pour son utilisation dans la prévention et/ou le traitement de l'alopécie.

Dans un mode de réalisation particulier de l'invention, l'alopécie est choisie dans le groupe constitué par l'alopécie androgénétique, l'alopécie post-ménopausique, l'alopécie réactionnelle et l'alopécie aerata.

Selon un aspect non couvert par l'invention, l'extrait selon l'invention ou la composition dermatologique selon l'invention peut être utilisé(e) en cosmétique pour des soins des cheveux et/ou du cuir chevelu, pour limiter la chute des cheveux, et/ou pour promouvoir la croissance capillaire et/ou pour augmenter la densité des follicules pileux et/ou pour obtenir une chevelure plus couvrante et/ou pour favoriser la régénération folliculaire, et/ou pour réduire la séborrhée du cuir chevelu, et/ou rendre les cheveux moins gras.

L'extrait selon l'invention ou la composition dermatologique selon l'invention sera avantageusement administré par voie topique et/ou par voie orale.
Une composition selon l'invention telle que décrite ici est caractérisée en ce qu'elle se présente sous une forme propre à une application topique au niveau du cuir chevelu et/ou des cheveux.
Une composition selon l'invention telle que décrite ici est caractérisée en ce qu'elle se présente sous une forme propre à une administration orale.

L'extrait selon l'invention ou la composition dermatologique selon l'invention permet ainsi d'enrayer la chute du cheveu, d'en prolonger son cycle, de sorte que le capital cheveu est préservé, en quantité et en qualité.

Les exemples qui suivent sont destinés à illustrer l'invention sans en limiter la portée. Ils sont donnés à titre indicatif non limitatif.

### EXEMPLES

### Préparation d'un extrait utilisé pour l'invention

### Exemple 1 : Extraction à reflux à l'éthanol 90

2,5 kilogrammes de parties aériennes sèches broyées de Lespedeza capitata sont extraites à reflux sous agitation par 25 Litres d'un mélange éthanol / eau 90:10 (v/v) pendant 1 heure dans un réacteur. L'extrait est ensuite filtré sur K900 et le solvant est évaporé de manière à obtenir 250 grammes d'une poudre verte avec un rendement massique de 10 %. L'extrait sec obtenu contient 26 % de polyphénols exprimés en épicatéchine.

### Exemple 2 : Extraction à reflux à l'acétate d'éthyle

100 grammes de parties aériennes sèches broyées de Lespedeza capitata sont extraites à reflux sous agitation par 1 Litre d'acétate d'éthyle pendant 1 heure dans un réacteur. L'extrait est ensuite filtré sur K900 et le solvant est évaporé de manière à obtenir 2 grammes d'une pâte verte avec un rendement massique de 2 %. L'extrait sec obtenu contient 3 % de polyphénols exprimés en épicatéchine.

### Exemple 3 : Extraction à température ambiante à l'éthanol 30

20 grammes de parties aériennes sèches broyées de Lespedeza capitata sont extraites à température ambiante sous agitation par 350 millilitres d'un mélange éthanol / eau 30:70 (v/v) pendant 12 heures dans un réacteur. L'extrait est ensuite filtré sur K900 et le solvant est évaporé de manière à obtenir 3,4 grammes d'une poudre marron avec un rendement massique de 17 %. L'extrait sec obtenu contient 36 % de polyphénols exprimés en épicatéchine.

### Exemple 4 : Extraction à l'éthanol 90 assistée par ultra-sons suivie d'une décoloration au charbon actif

26 grammes de parties aériennes sèches broyées de Lespedeza capitata sont mis en contact avec 260 millilitres d'un mélange éthanol / eau 90:10 (v/v) puis extraits sous l'action des ultra-sons (20 kHz) pendant 3 fois 1 minute à une amplitude de 100 %. Après filtration sur K900, l'extrait est concentré puis décoloré au charbon actif (0,2% p/v). Après filtration, le solvant est évaporé de manière à obtenir 1,5 grammes d'une poudre marron avec un rendement massique de 6 %. L'extrait sec obtenu contient 25 % de polyphénols exprimés en épicatéchine.

### Exemple 5 : Extraction à reflux à l'éthanol 90 et décoloration au charbon actif

280 grammes de parties aériennes sèches broyées de Lespedeza capitata sont extraites à reflux sous agitation par 2800 millilitres d'un mélange éthanol / eau 90:10 (v/v) pendant 1 heure dans un réacteur. Après filtration sur K900, l'extrait est concentré puis décoloré au charbon actif (0,2% p/v). Après filtration, le solvant est évaporé de manière à obtenir 25 grammes d'extrait sous forme d'une poudre marron avec un rendement massique de 9 %. L'extrait obtenu contient 25 % de polyphénols exprimés en épicatéchine.

### Exemple 6 : Extraction à reflux à l'éthanol 90 suivie d'une décoloration au charbon actif et mise sur support

2,5 kilogrammes de parties aériennes sèches broyées de Lespedeza capitata sont extraites à reflux sous agitation par 25 Litres d'un mélange éthanol / eau 90:10 (v/v) pendant 1 heure dans un réacteur. Après filtration sur K900, l'extrait est concentré puis décoloré au charbon actif (0,2% p/v). Après filtration, l'extrait est séché sur 1,2-pentanediol de façon à obtenir 46 grammes d'extrait sous forme d'un liquide visqueux sombre. L'extrait obtenu contient 50% de 1,2-pentanediol et 12 % de polyphénols exprimés en épicatéchine.

### Exemple 7 : Extraction à reflux à l'éthanol 90

100 grammes de feuilles sèches broyées de Lespedeza capitata sont extraites à reflux sous agitation par 1 Litre d'un mélange éthanol / eau 90:10 (v/v) pendant 1 heure dans un réacteur. L'extrait est ensuite filtré sur K900 et le solvant est évaporé de manière à obtenir 17 grammes d'une poudre verte avec un rendement massique de 17 %. L'extrait sec obtenu contient 28 % de polyphénols exprimés en épicatéchine.

### Evaluation pharmacologique

### Exemple 8 : inhibition de la synthèse et la libération de DKK1 par les cellules de la papille dermique par un extrait de partie aérienne de Lespedeza capitata

L'objectif de cette étude est d'évaluer l'effet d'un extrait selon la présente invention sur la production et la sécrétion de DKK1 par les cellules papillaires dermiques du follicule pileux humain et d'évaluer l'intérêt dermo-cosmétique de cet extrait en tant qu'agent antichute. En effet l'expression de DKK1 a été démontrée comme un facteur clef de l'alopécie androgénique (cf Kwack et al, BMB Reports, Volume 43, Issue 10,2010, pp.688-692)

Les expériences ont été effectuées sur des cellules papillaires dermiques du follicule pileux humain provenant de trois donneurs différents.

Les cellules sont ensemencées en plaques de 96 puits et mises en culture pendant 24 heures dans des milieux de culture standards. Le milieu est ensuite remplacé par un milieu d'essai contenant ou pas (condition contrôle) le composé à tester. L'extrait de Lespedeza capitata est un extrait préparé selon l'exemple 5 de la présente invention, cet extrait est testé à deux concentrations 3 µg/ml et 10 µg/ml, il s'agit de concentration d'extrait sec, dilué dans du DMSO. Vingt-quatre heures après les surnageants de culture sont recueillis et stockés à -80°C.

La protéine DKK1 a été quantifiée par Luminex (référence du kit : HBNMAG-51K, Millipore) conformément aux instructions du fabricant.

Les données brutes sont analysées par les logiciels Microsoft Excel et Prism. L'analyse statistique est réalisée grâce à des comparaisons inter-groupes par une ANOVA suivie par un test de Dunnett.

### Résultats

Le tableau 1 ci-dessous représente l'expression de DKK1 en présence ou non d'extrait de Lespedeza capitata et son pourcentage d'inhibition

| | conc | Moy [DKK1] pg/ml | sem | % inh | Stats |
|---|---|---|---|---|---|
| Contrôle | - | 145.9 | 19.2 | - | - |
| Extrait de Lespedeza | 3µg/ml | 104.2 | 11.4 | 28 | P<0.01 |
| | 10µg/ml | 40.5 | 5.2 | 72 | P<0.01 |

| | | | | | |
|---|---|---|---|---|---|
| Con : concentration ; Moy : moyenne ; sem : erreur standard à la moyenne ; % inh : % d'inhibition par rapport à la condition contrôle ; Stats : statistique. | | | | | |

Les cellules papillaires dermiques du follicule humain sécrètent naturellement la protéine DKK1, de l'ordre de 150 pg/ml. Lorsque ces cellules sont traitées par un extrait de Lespedeza capitata, l'expression de cette protéine est réduite de façon concentration-dépendante et significativement dès la concentration de 3 µg/ml. En présence de 10 µg/ml d'extrait de Lespedeza capitata la réduction d'expression de DKK1 atteint 72%.

Les inventeurs mettent ainsi en évidence qu'un extrait de Lespedeza capitata présente une activité inhibitrice sur la cible DKK1.

Cet extrait présente donc une activité intéressante pour son utilisation comme agent actif dans la prévention et/ou le traitement de la chute des cheveux.

### Exemple 9 : inhibition de l'activité de la 5α-réductase dans des cellules de la papille dermique par un extrait de partie aérienne de Lespedeza capitata

L'objectif de cette étude est d'évaluer l'effet d'un extrait selon la présente invention sur l'activité de la 5α-réductase dans des cellules de la papille dermique afin de mesurer l'intérêt de cet extrait dans le domaine de l'antichute et/ou de la séborrhée du cuir chevelu.

Les expériences ont été effectuées sur des cellules papillaires dermiques du follicule pileux humain provenant de deux donneurs différents. Les cellules sont ensemencées (150 000 cellules par puits) en plaques de 24 puits et mises en culture pendant 24 heures dans des milieux de cultures standards. Le milieu est ensuite remplacé par un milieu d'essai contenant ou pas (condition contrôle) le composé à tester ou le produit de référence (finastéride à 10 µM) pendant 24 heures. L'extrait de Lespedeza capitata est un extrait préparé selon l'exemple 5 de la présente invention, cet extrait est testé à deux concentrations 30 µg/ml et 100 µg/ml, il s'agit de concentration d'extrait sec, dilué dans du DMSO. Puis les cellules sont traitées pendant 24 heures par un milieu contenant de la testostérone C¹⁴ et contenant ou pas les composés à tester. Les surnageants de culture sont ensuite recueillis pour des analyses du métabolisme de la testostérone.

Les molécules stéroïdes des surnageants sont extraites par un mélange Chloroforme/Méthanol. La phase organique est recueillie et le DHT est séparé par chromatographie sur couche mince et en utilisant un système de solvant contenant du dichlorométhane, de l'éthylacétate et du méthanol. Une autoradiographie est ensuite réalisée sur la chromatographie et la testostérone transformée est estimée par analyses densitométriques. Les données brutes sont analysées par le logiciel Excel de Microsoft. L'analyse statistique est réalisée grâce à des comparaisons inter-groupes par une ANOVA à mesures répétées suivies par un test de Dunnett.

Dans cette étude le ratio DHT/testostérone est sélectionné pour représenter l'activité enzymatique 5α-réductase. Si ce ratio diminue avec l'ajout d'un composé, ce composé est alors considéré comme un inhibiteur de la 5α-réductase. Pour être sûr que ce composé est vraiment spécifique de cette enzyme, il est important de vérifier une diminution du ratio 5α-androstane-3α, 17β-diol / testostérone, du ratio androstenedione / testostérone et une augmentation du ratio 4-androstene-3,17-dione / testostérone.

### Résultats

Le tableau 2 ci-dessous représente l'activité de la 5α-réductase (ratio DHT/testostérone) après une incubation de 24h + 24h avec le composé de référence (finastéride) et l'extrait de Lespedeza capitata

| | Conc | Moy | sem | % inh | sem | stats |
|---|---|---|---|---|---|---|
| Contrôle | - | 0,31 | 0,10 | 0.0 | 7.2 | - |
| Finastéride | 10µM | 0,02 | 0,01 | 94.2 | 1.4 | P<0,01 |
| Extrait de Lespedeza | 30µg/ml | 0,23 | 0,05 | 21.8 | 8.6 | NS |
| | 100µg/ml | 0,11 | 0,01 | 59.6 | 9.0 | P<0,05 |

| | | | | | | |
|---|---|---|---|---|---|---|
| Con : concentration ; Moy : moyenne ; sem : erreur standard à la moyenne ; % inh : % d'inhibition par rapport à la condition contrôle ; stats : statistique. | | | | | | |

Le traitement des cellules papillaires dermiques du follicule pileux humain avec le finastéride montre une très forte réduction de la conversion de testostérone en DHT, (diminution du ratio DHT/testostérone). Ces résultats étaient attendus et permettent de valider le test.

En présence d'extrait de Lespedeza capitata, le ratio DHT/testostérone est réduit de façon concentration-dépendante, cette réduction (qui atteint 60%) est statistiquement significative avec une concentration de Lespedeza capitata à 100µg/ml. Bien que l'activité à 30µg/ml n'ait pas été qualifiée de statistiquement significative elle est pour autant tout aussi démonstrative.

Les inventeurs ont ainsi clairement mis en évidence qu'un extrait de Lespedeza capitata présente une activité inhibitrice sur la 5α-réductase.

Cet extrait de Lespedeza capitata présente donc une activité intéressante pour son utilisation comme agent actif dans la prévention et/ou le traitement des troubles dermo-capillaires, en particulier la chute des cheveux et/ou de la séborrhée du cuir chevelu et troubles associés.

### Exemple 10 : Effet sur l'activité métabolique et/ou de prolifération de cellules de la papille dermique par un extrait de partie aérienne de Lespedeza capitata

La papille dermique est localisée à la base du follicule pileux, profondément ancré dans le tissu cutané. On sait depuis longtemps que les cellules des papilles dermiques, jouent un rôle clé dans la croissance du follicule pileux. Hautement vascularisée, la papille dermique assure la fonction de nutrition pour le cheveu et fournit l'information moléculaire nécessaire pour la prolifération et la différenciation des cellules épithéliales pour produire un nouveau cheveu à chaque cycle. Des études ont montré que sur des follicules pileux humains sains et lors d'expériences chez le rat, dans un modèle de régénération de moustaches induite par lésions, il existe une corrélation entre la taille de la papille dermique (et aussi avec le nombre de cellules de la papille dermique et la taille de la tige pilaire). Cette corrélation est maintenue dans le contexte d'une perte progressive des cheveux, dans laquelle la taille des follicules pileux et des tiges pilaires est réduite successivement avec les cycles de croissance des cheveux. Par ailleurs, l'augmentation du nombre de cellules dans la papille dermique dans un follicule peut se produire en partie par le recrutement de nouvelles cellules à la papille dermique, mais la prolifération de cellules de la papille dermique peut aussi contribuer à cette expansion.

L'objectif de cette étude est d'évaluer l'effet d'un extrait selon la présente invention sur l'activité métabolique et/ou la prolifération des cellules de la papille dermique afin de mesurer l'intérêt de cet extrait dans le domaine de l'antichute.

Les expériences ont été effectuées sur des cellules papillaires dermiques du follicule pileux humain provenant de trois donneurs différents.

Les cellules sont ensemencées en plaques de 96 puits et mises en culture pendant 24 heures dans des milieux de culture standards. Le milieu est ensuite remplacé par un milieu d'essai (milieu FFDPC, Follicle Dermal Papilla Cell Basal Medium, Ref C-26500 de chez Promocell) contenant ou pas (condition contrôle) le composé à tester. Un groupe avec le même milieu mais supplémenté à 5% de milieu complémentaire (Follicle Dermal Papilla Cell Growth Medium SuupplementPaxk, Ref C-39620, Promocell) permet d'avoir un contrôle positif. L'extrait de Lespedeza capitata est un extrait préparé selon l'exemple 2 de la présente invention, cet extrait est testé à deux concentrations 3 µg/ml et 10 µg/ml, il s'agit de concentration d'extrait sec, dilué dans du DMSO. Soixante-douze heures après les surnageants de culture sont recueillis et stockés à -80°C.

L'activité métabolique cellulaire et la prolifération sont évaluées par une méthode colorimétrique, le sel soluble de tetrazolium (WIST-1), qui est rouge clair devient rouge quand il est réduit en formazan (test commercialisé par TakaRa Colntech, sous le numéro catalogue MK400). Les données brutes sont analysées par le logiciel Excel de Microsoft. L'analyse statistique est réalisée grâce à des comparaisons inter-groupes par une ANOVA à mesures répétées suivies par un test de Dunnett

### Résultats

Le tableau 3 montre les résultats de l'extrait de Lespedeza capitata sur l'activité métabolique et la prolifération cellulaire.

| | Concentration | Stim (%) | sem | stats |
|---|---|---|---|---|
| | | | | |
| Contrôle | - | 0 | 0,15 | - |
| Mil suppl | 5% | 60 | 28 | P<0,05 |
| Extrait de Lespedeza | 3 µg/ml | 51 | 4,3 | P<0.05 |
| | 10 µg/ml | 44 | 14,3 | P=NS |

| | | | | |
|---|---|---|---|---|
| Mil Suppl : milieu avec supplément, permettant une prolifération cellulaire ; Moy : moyenne ; sem : erreur standard à la moyenne ; % stim : % de stimulation par rapport à la condition contrôle ; stats : statistique ; DO : densité optique. | | | | |

Le traitement des cellules de la papille dermique par un milieu supplémenté induit une activation significative de la prolifération cellulaire et/ou de l'activité métabolique, ce résultat permet de valider ce modèle. L'extrait de Lespedeza capitata à 3µg/ml induit une augmentation statistiquement significative de la prolifération cellulaire et/ou de l'activité métabolique. Cette activation n'atteint pas la significativité lorsque l'extrait de Lespedeza capitata est à 10µg/ml, sans doute en raison d'une forte variabilité des résultats, mais elle n'en est pas pour le moins démonstrative de l'effet de l'extrait.
Les inventeurs ont ainsi clairement mis en évidence qu'un extrait de Lespedeza capitata présente une activité stimulatrice sur la prolifération cellulaire et/ou l'activité métabolique. Ainsi, cet extrait de Lespedeza capitata présente donc une activité intéressante pour son utilisation en promouvant la croissance des cheveux, en renforçant et stimulant le cycle de vie des cheveux et en promouvant la régénération capillaire.

### Exemple 11 : Evaluation d'un extrait de partie aérienne de Lespedeza capitata sur l'inhibition de JAK1, JAK2 et JAK3 humaines

JAK-STAT est une voie de signalisation de transduction régulant la croissance, la survie, la différenciation et la résistance aux pathogènes. Cette voie médie les effets des cytokines, des interférons et des facteurs de croissance. Chez les mammifères la famille JAK est constituée de quatre membres : JAK1, JAK2, JAK3 et TYK2. Il est montré dans une étude réalisée sur des follicules pileux de souris, que la voie JAK-STAT est dynamiquement régulée dans le cycle capillaire ; en effet la voie JAK-STAT est activée lors des phases catagène et télogène et réprimée au début de la phase anagène. De plus il est démontré chez la souris, qu'un traitement topique en phase télogène avec des inhibiteurs de la voie JAK-STAT, incluant le Tofacitinib (JAK1/3 > JAK2 > TYK2) et le Ruxolitinib (JAK1/2 > TYK2 > JAK3), résultait en une réentrée rapide en début de phase anagène (Harel et al., 2015 Sci. Adv.1,e1500973). Dans cette même étude il est également montré que l'inhibition de JAK-STAT dans des follicules pileux humains augmente la croissance du cheveu ex vivo. Ces données suggèrent que la voie de signalisation JAK-STAT pourrait être une nouvelle cible pour stimuler la croissance des cheveux.

Le but de cet exemple est d'évaluer si un extrait selon la présente invention peut inhiber la voie de signalisation JAK-STAT. Cette inhibition est évaluée sur des protéines recombinantes humaines JAK1, JAK2 ou JAK3.

### Méthodes

L'extrait de Lespedeza capitata est un extrait préparé selon l'exemple 5 de la présente invention, cet extrait est dilué dans du DMSO, il est testé à diverses concentrations (0,1 - 1000 µg/ml). Les contrôles positifs (Tofacitinib et Ruxolitinib) sont également évalués dans ce test.

Les produits à tester sont incubés avec une protéine recombinante humaine JAk1, JAK2 ou JAK 3 avec un tampon réactionnel (Tris/HCl pour JAK1, MOPS (acide 3-morpholino-1-propanesulfonique) pour JAK2 et JAK3), de l'EDTA et les substrats peptidiques spécifiques. La réaction de phosphorylation est ensuite initiée par l'addition d'un mélange d'acétate de magnésium et d'ATP radiomarqué (45µM pour JAK1 et JAK2 et 10µM pour JAK3). Après incubation pendant 40 minutes à température ambiante, la réaction est stoppée par ajout d'acide phosphorique. Quatre lavages avec l'acide phosphorique et un avec le méthanol sont réalisés pour éluer les petites molécules dont l'ATP marqué. Enfin la radioactivité du substrat phosphorylé spécifique est comptée. Les composés sont testés sur trois expériences distinctes et pour chaque expérience, des doublons sont réalisés.

Les valeurs de CI₅₀, en µg/ml, de chaque protéine recombinante JAK1, JAK2 et JAK3 en fonction du produit testé incubé sont présentées dans le tableau 4 suivant :

| Composés | JAK1 | JAK2 | JAK3 |
|---|---|---|---|
| | CI₅₀ (µg/ml) | CI₅₀ (µg/ml) | CI₅₀ (µg/ml) |
| Ruxolitinib | 0,00037 | 0,00023 | 0,0060 |
| Tofacitinib | 0,00087 | 0,00367 | 0,00057 |
| Extrait Lespedeza capitata | 24,8 | 146,5 | 3,9 |

Les résultats obtenus avec les composés de référence (Ruxolitinib et Tofacitinib) sont conformes à ceux attendus. En effet, ces deux composés inhibent fortement l'activité JAK-STAT (entre 97% et 100% d'inhibition maximale, avec des valeurs de CI₅₀ très faibles, de l'ordre du nanogramme par millilitre). Ruxolitinib montre une affinité plus importante pour JAK1 ou JAK2 que pour JAK3. Alors que Tofacitinib a plus d'affinité pour JAK1 ou JAK3 que pour JAK2. Ces résultats attendus valident ce test.

L'extrait de partie aérienne de Lespedeza capitata montre une forte inhibition de JAK1 (100% d'inhibition maximale) avec une valeur de CI₅₀ de 24,8 µg/ml, une forte inhibition de JAK2 (100% d'inhibition maximale) avec une valeur de CI₅₀ un peu plus élevée de 146,5 µg/ml, et également une forte inhibition pour JAK3 (100% d'inhibition maximale) avec une valeur de CI₅₀ de 3,9 µg/ml.

Ce test démontre que l'extrait de Lespedeza capitata induit une inhibition de l'activité tyrosine kinase (avec une affinité plus forte pour JAK3), dévoilant une activité pharmacologique d'intérêt pour promouvoir la croissance capillaire.

### Exemple 12 : Confirmation de l'activité inhibitrice de la voie JAK-STAT d'un extrait de partie aérienne de Lespedeza capitata au niveau cellulaire

Le but de cet exemple est de confirmer l'activité inhibitrice d'un extrait de Lespedeza capitata de la voie de signalisation JAK-STAT sur un modèle cellulaire, les kératinocytes folliculaires de la gaine épithéliale externe de follicule pileux (modèle ORS Outer Root Sheath). Dans ce modèle, l'interleukine IL-6 est utilisée pour activer la voie JAK1/2-STAT3 par l'activation des récepteurs d'IL-6 et du GP130 qui sont tous les deux exprimés au niveau de cette gaine épithéliale des follicules pileux. II-6 est une cytokine qui agit comme un inhibiteur du cycle de croissance du cheveu, sa surexpression dans un modèle de souris transgénique entraine une croissance du cheveu retardée. Par ailleurs, dans l'alopécie androgénétique IL-6 serait surexprimée dans les cellules de la papille dermique sous l'influence des androgènes (Kwack et al., 2012, J Invest Dermatology 132(1) : 43-9). Il a également été reporté qu'IL-6 retarde la croissance du follicule pileux chez l'homme.

### Méthode

L'étude est réalisée sur des kératinocytes folliculaires de la gaine épithéliale externe de follicule pileux (modèle ORS Outer Root Sheath). Les kératinocytes sont mis en culture en plaque de 96 puits dans un milieu adéquat (CnT-PR de CelInTech). Vingt-quatre heures après, les cellules sont lavées avec un tampon phosphate alcalin (PBS) et le milieu est changé par le milieu CnT-PR-H (milieu de maintien standard pour des kératinocytes primaires humains). Après 48 heures, les cellules sont traitées pendant 1 heure avec les composés à tester (extrait de Lespedeza capitata à 30 et 100µg/ml dilué dans du DMSO) et les composés de référence (Ruxolitinib et Tofacitinib à 5µM tous les deux, dilués dans du DMSO). L'extrait de Lespedeza capitata testé est un extrait préparé selon l'exemple 5 de la présente invention. Puis le traitement de stimulation par IL-6 est réalisé à 100ng/ml pendant 15 minutes.

Toutes les conditions sont testées sur des cellules de n=3 donneurs distincts. Les extraits de Lespedeza capitata sont testés sur n=3 expériences distinctes, les conditions contrôles, de stimulation et avec les composés de référence sont réalisés sur n=6 expériences. Pour chaque expérience, les données sont réalisées en triple exemplaire.

L'activité de JAK est estimée par l'évaluation du niveau de phosphorylation de la protéine STAT3. L'analyse statistique est réalisée par un test paramétrique après vérification de la normalité et de l'équivalence de la variance, sinon un test non-paramétrique est choisi.

### Résultats

Les résultats sont résumés dans le tableau 5 suivant :

| Condition | Groupes | % de STAT phosphorylé | | |
|---|---|---|---|---|
| | | Moyenne | esm | % inh |
| - | Contrôle | 100 | 0,0 | 100 |
| IL-6 | Contrôle | 116 | 1,0 | 0 |
| IL-6 | Ruxolitinib | 102 | 3,5 | 85 |
| IL-6 | Tofacitinib | 99 | 2,4 | 104 |
| IL-6 | Lespedeza capitata (30µg/ml) | 71 | 7,2 | 285 |
| IL-6 | Lespedeza capitata (100µg/ml) | 59 | 3,7 | 366 |

| | | | | |
|---|---|---|---|---|
| Esm : erreur standard à la moyenne ; % inh : pourcentage d'inhibition. | | | | |

Le traitement des kératinocytes avec l'IL-6 induit une augmentation significative du niveau de phosphorylation de STAT3, cette augmentation atteint 16% et est statistiquement significative (p<0,05). Les composés de référence (Ruxolitinib et Tofacitinib testés à 5µM) réduisent significativement le niveau de phosphorylation de STAT3, respectivement de 85% et 104%, p<0,05 pour Ruxolitinib et p<0,01 pour Tofacitinib. Ces résultats attendus valident le test.

L'extrait de Lespedeza capitata aux deux concentrations testées inhibe significativement (p<0,01 pour les deux concentrations) et de façon très marquée le niveau de phosphorylation de STAT3 induit par la stimulation par IL-6.

Les inventeurs mettent ainsi en évidence l'intérêt d'un extrait de partie aérienne de Lespedeza capitata dans la promotion de la repousse capillaire.

## Revendications

1. Extrait de *Lespedeza capitata* pour son utilisation pour le traitement et/ou la prévention d'au moins un trouble dermo-capillaire, **caractérisé en ce que** le au moins un trouble dermo-capillaire est choisi parmi l'alopécie, la séborrhée du cuir chevelu, et leur combinaison et que l'extrait est obtenu à partir d'une ou plusieurs parties de plante *Lespedeza capitata* choisie parmi les parties aériennes telles que tiges, branches, feuilles, fruits, graines et/ou fleurs suite à une extraction par solvant hydrophile ou moyennement polaire choisi dans le groupe constitué des alcools en C1 à C5, des esters d'alkyles et un mélange hydro-alcoolique.

2. Extrait de *Lespedeza capitata* pour son utilisation selon la revendication 1, **caractérisé en ce que** le solvant d'extraction est un mélange éthanol/eau.

3. Extrait de *Lespedeza capitata* pour son utilisation selon l'une quelconque des revendications 1 à 2, **caractérisé en ce que** l'extrait comprend de 2 à 40 % en poids de polyphénols totaux par rapport au poids de l'extrait sec.

4. Extrait de *Lespedeza capitata* pour son utilisation selon l'une quelconque des revendications 1 à 3, **caractérisé en ce que** l'alopécie est choisie dans le groupe constitué par l'alopécie androgénétique, l'alopécie réactionnelle, l'alopécie post-ménopausique et l'alopécie aerata.

5. Composition dermatologique pour son utilisation pour le traitement et/ou la prévention d'au moins un trouble dermo-capillaire choisi parmi l'alopécie, la séborrhée du cuir chevelu, et leur combinaison comprenant à titre d'agent actif un extrait de Lespedeza capitata tel que défini dans l'une quelconque des revendications 1 à 3, avec au moins un excipient dermatologiquement acceptable.

6. Composition pour son utilisation selon la revendication 5, **caractérisée en ce qu'**elle comprend 0,0001% à 2%, de préférence 0,001 à 1% en poids, d'extrait de Lespedeza capitata, en poids d'extrait sec par rapport au poids total de la composition.

7. Composition pour son utilisation selon l'une des revendications 5 à 6, **caractérisée en ce qu'**elle se présente sous une forme propre à une application topique au niveau du cuir chevelu et/ou des cheveux.

8. Composition pour son utilisation selon l'une des revendications 5 à 6, **caractérisée en ce qu'**elle se présente sous une forme propre à une administration orale.

9. Composition pour son utilisation selon l'une quelconque des revendications 5 à 8, **caractérisée en ce que** l'alopécie est choisie dans le groupe constitué par l'alopécie androgénétique, l'alopécie réactionnelle, l'alopécie post-ménopausique et l'alopécie aerata.

## Patentansprüche

1. *Lespedeza-capitata-Extrakt* zur Verwendung bei der Behandlung und/oder der Prävention mindestens einer Haut-Haar-Erkrankung, **dadurch gekennzeichnet, dass** die mindestens eine Haut-Haar-Erkrankung ausgewählt ist aus Alopezie, Seborrhoe der Kopfhaut und ihrer Kombination und dass der Extrakt ausgehend von einem oder mehreren Teilen der Pflanze *Lespedeza capitata,* der bzw. die ausgewählt ist bzw. sind aus den oberirdischen Teilen, wie Stängeln, Zweigen, Blättern, Früchten, Samen und/oder Blüten, infolge einer Extraktion durch ein hydrophiles oder mittelpolares Lösungsmittel erhalten wird, das ausgewählt ist aus der Gruppe, die besteht aus C1- bis C5-Alkoholen, Alkylestern und einem hydroalkoholischen Gemisch.

2. *Lespedeza-capitata-Extrakt* zur Verwendung nach Anspruch 1, **dadurch gekennzeichnet, dass** das Extraktionslösungsmittel ein Ethanol/Wasser-Gemisch ist.

3. *Lespedeza-capitata-Extrakt* zur Verwendung nach einem der Ansprüche 1 bis 2, **dadurch gekennzeichnet, dass** der Extrakt, bezogen auf das Gewicht des Trockenextrakts, zwischen 2 und 40 Gew.-% Gesamtpolyphenole umfasst.

4. *Lespedeza-capitata-Extrakt* zur Verwendung nach einem der Ansprüche 1 bis 3, **dadurch gekennzeichnet, dass** die Alopezie ausgewählt ist aus der Gruppe, die besteht aus androgenetischer Alopezie, reaktioneller Alopezie, Postmenopausenalopezie und Alopecia aerata.

5. Dermatologische Zusammensetzung zur Verwendung bei der Behandlung und/oder der Prävention mindestens einer Haut-Haar-Erkrankung, die ausgewählt ist aus Alopezie, Seborrhoe der Kopfhaut und ihrer Kombination, die als Wirkstoff einen Lespedeza-capitata-Extrakt nach einem der Ansprüche 1 bis 3 mit mindestens einem dermatologisch annehmbaren Hilfsstoff umfasst.

6. Zusammensetzung zur Verwendung nach Anspruch 5, **dadurch gekennzeichnet, dass** sie, in Trockenextraktgewicht bezogen auf das Gesamtgewicht der Zusammensetzung, 0,0001 bis 2, vorzugsweise 0,001 bis 1 Gew.-%, Lespedeza-capitata-Extrakt umfasst.

7. Zusammensetzung zur Verwendung nach einem der Ansprüche 5 bis 6, **dadurch gekennzeichnet, dass** sie eine Form aufweist, die sich zu einer topischen Anwendung im Bereich der Kopfhaut und/oder der Haare eignet.

8. Zusammensetzung zur Verwendung nach einem der Ansprüche 5 bis 6, **dadurch gekennzeichnet, dass** sie eine Form aufweist, die sich zu einer oralen Verabreichung eignet.

9. Zusammensetzung zur Verwendung nach einem der Ansprüche 5 bis 8, **dadurch gekennzeichnet, dass** die Alopezie ausgewählt ist aus der Gruppe, die besteht aus androgenetischer Alopezie, reaktioneller Alopezie, Postmenopausenalopezie und Alopecia aerata.

## Claims

1. An extract of *Lespedeza capitata* for its use for the treatment and/or prevention of at least one dermo-capillary disorder, **characterized in that** the at least one dermo-capillary disorder is selected from alopecia, seborrhea of the scalp, and a combination thereof and that the extract is obtained from one or more parts of the *Lespedeza capitata* plant selected from aerial parts such as stems, branches, leaves, fruits, seeds and/or flowers following an extraction by a hydrophilic or moderately polar solvent selected from the group consisting of C1 to C5 alcohols, alkyl esters and a hydro-alcoholic mixture.

2. The extract of *Lespedeza capitata* for its use according to claim 1, **characterized in that** the extraction solvent is an ethanol/water mixture.

3. The extract of *Lespedeza capitata* for its use according to any one of claims 1 to 2, **characterized in that** the extract comprises from 2 to 40% by weight of total polyphenols relative to the weight of the dry extract.

4. The extract of *Lespedeza capitata* for its use according to any one of claims 1 to 3, **characterized in that** the alopecia is selected from the group consisting of androgenetic alopecia, reactive alopecia, post-menopausal alopecia and alopecia aerata.

5. A dermatological composition for its use for the treatment and/or prevention of at least one dermo-capillary disorder selected from alopecia, seborrhea of the scalp, and a combination thereof comprising as active agent an extract of Lespedeza capitata as defined in any one of claims 1 to 3, with at least one dermatologically acceptable excipient.

6. The composition for its use according to claim 5, **characterized in that** it comprises 0.0001% to 2%, preferably 0.001 to 1% by weight, of extract of Lespedeza capitata, by weight of dry extract relative to the total weight of the composition.

7. The composition for its use according to one of claims 5 to 6, **characterized in that** it is in a form suitable for topical application to the scalp and/or hair.

8. The composition for its use according to one of claims 5 to 6, **characterized in that** it is in a form suitable for oral administration.

9. The composition for its use according to any one of claims 5 to 8, **characterized in that** the alopecia is selected from the group consisting of androgenetic alopecia, reactive alopecia, post-menopausal alopecia and alopecia aerata.
